# EUROPÄISCHE PATENTSCHRIFT

(11) **EP 3 442 525 B1**
(45) Veröffentlichungstag und Bekanntmachung des Hinweises auf die Patenterteilung: **17.07.2024**
(21) Anmeldenummer: 17749110.7
(22) Anmeldetag: 18.04.2017
(51) Int. Cl.: A61K 31/4045, A61P 25/08, A61P 25/16, A61P 25/20, A61P 25/00

(54) **MELATONIN ZUR BEHANDLUNG EINER REM-SCHLAFVERHALTENSSTÖRUNG EINES MENSCHEN**
MELATONIN FOR THE TREATMENT OF REM SLEEP BEHAVIOUR DISORDER IN HUMANS
MÉLATONINE POUR LE TRAITEMENT DE TROUBLES DU COMPORTEMENT EN SOMMEIL PARADOXAL D'UN HUMAIN

(30) Priorität: 14.04.2016 DE 102016004397
(43) Veröffentlichungstag der Anmeldung: 20.02.2019
(73) Patentinhaber: Kunz, Dieter, 14163 Berlin (DE)
(72) Erfinder: Kunz, Dieter, 14163 Berlin (DE)
(74) Vertreter: Kayser, Christoph
(86) Internationale Anmeldenummer: PCT/DE2017/000104
(87) Internationale Veröffentlichungsnummer: WO 2017/177999

(56) Entgegenhaltungen:
- WO-A1-2011/150098
- US-A- 5 958 964
- DIETER KUNZ ET AL: "Melatonin as a therapy in rem sleep behavior disorder patients: An open-labeled pilot study on the possible influence of melatonin on rem-sleep regulation", MOVEMENT DISORDERS., vol. 14, no. 3, 1 May 1999 (1999-05-01), US, pages 507 - 511, XP055406831, ISSN: 0885-3185, DOI: 10.1002/1531-8257(199905)14:3<507::AID-MDS1021>3.0.CO;2-8
- KENJIRO ONO ET AL: "Effect of melatonin on [alpha]-synuclein self-assembly and cytotoxicity", NEUROBIOLOGY OF AGING, vol. 33, no. 9, 1 September 2012 (2012-09-01), US, pages 2172 - 2185, XP055406687, ISSN: 0197-4580, DOI: 10.1016/j.neurobiolaging.2011.10.015
- DATABASE WPI Week 200760, Derwent World Patents Index; AN 2007-629202, XP002773860
- SEONGHAN KIM ET AL: "[alpha]-Synuclein, Parkinson's disease, and Alzheimer's disease", PARKINSONISM AND RELATED DISORDERS, vol. 10, 1 May 2004 (2004-05-01), GB, pages S9 - S13, XP055406654, ISSN: 1353-8020, DOI: 10.1016/j.parkreldis.2003.11.005
- MARIA J. MARTI' ET AL: "Clinical Overview of the Synucleinopathies", MOVEMENT DISORDERS., vol. 18, no. S6, 1 September 2003 (2003-09-01), US, pages 21 - 27, XP055360522, ISSN: 0885-3185, DOI: 10.1002/mds.10560

## Beschreibung

Die vorliegende Erfindung betrifft Melatonin zur Verwendung bei einer Behandlung einer REM-Schlafverhaltensstorung eines Menschen mit einem individuellen Chronotypus.

Die Erfindung ist in den nachfolgen Patentansprüchen offenbart.

Die REM-Schlafverhaltensstörung (englisch: REM Sleep Behavior Disorder - RBD) imponiert klinisch durch das Ausagieren von Traumerleben. Die Träume sind zumeist lebhaft und aggressiv, führen nicht selten zu auch schweren Verletzungen der Patienten selbst oder der Bettpartner. RBD ist kategorisiert innerhalb der Krankheitsgruppe der Parasomnien und definiert in der Internationalen Klassifikation der Schlafstörungen (ICSD-3) sowie dem Diagnostischen und Statistischen Manual psychischer Störungen (DSM-5).

Ursache und ursächliche Therapie sind bisher nicht bekannt. Die Standardbehandlung erfolgt bislang symptomatisch durch Clonazepam^{®}. Clonazepam^{®} reduziert die motorischen Phänomene in der ersten Nacht nach Applikation, nach Absetzen treten die Symptome zumeist in vollem Umfang in der ersten Nacht wieder auf. Clonazepam^{®} verursacht Nebenwirkungen, die gerade bei Patienten mit RBD gravierend sein können und nicht selten zum Absetzen der Medikation führen. Dies sind kognitive Beeinträchtigungen (unerwünscht bei dieser Patientengruppe, die häufig parallel auch unter dementiellen Syndromen leidet), Muskelrelaxation (unerwünscht, da bei gleichzeitigem nächtlichem Wasserlassen die Gefahr von Stürzen droht) sowie über die Muskelrelaxation eine Verstärkung von schlafbezogenen Atemstörungen mit deren Konsequenzen.

Die Verwendung von exogenem Melatonin ist aus DIETER KUNZ et al. bekannt: "Melatonin as a therapy in rem sleep behavior disorder patients: An open-labeled pilot study on the possible influence of melatonin on remsleep regulation", MOVEMENT DISORDER, Bd. 14, Nr.3, 1. Mai 1999 (1999-05-01), Seiten 507-511. Diese Studie geht allerdings nicht auf den individuellen Chronotypus der behandelten Patienten ein.

Befunde der letzten Jahre haben gezeigt, dass RBD früher Vorläufer für die neurodegenerative Erkrankungsgruppe der Alpha-Synukleinopathien ist. Bei einer Konversionsrate von 8 - 9 Prozent pro Jahr liegt die derzeit angenommene Wahrscheinlichkeit, innerhalb von 15 Jahren klinische Symptome einer neurodegenerativen Erkrankung zu entwickeln, bei über 80 Prozent. Diese Erkrankungsgruppe besteht im Wesentlichen aus den Erkrankungsbildern Lewy-Body-Demenz, multiple Systematrophie und Parkinson-Erkrankung. Es wird heute davon ausgegangen, dass die Mehrzahl der Patienten, die diese Gruppe von neurodegenerativen Erkrankungen erleidet, im Vorfeld bereits RBD-Zeichen aufwiesen. Ursache und ursächliche Therapie sind auch hier bislang unbekannt. Alpha-Synukleinopathien werden daher bislang symptomatisch behandelt. Auch hier geht die Behandlung zumeist mit erheblichen negativen Nebenwirkungen einher.

Vor 20 Jahren wurde durch den Erfinder erstmalig in einer nicht veröffentlichten Studie einer Gruppe von Patienten mit REM-Schlafverhaltensstörung mit Melatonin behandelt. In hohem Maße unerwartet, sistierten die Symptome fast vollständig. Der Therapieverlauf war in hohem Maße ungewöhnlich und entspricht in keiner Weise bekannten pharmakologischer Wirkmechanismen.

In Nacht 1 nach der Applikation von Melatonin waren keine Effekte zu erkennen. Erste Verbesserung der nächtlichen Symptomatik wurde innerhalb von zehn Tagen berichtet. Eine weitere Verbesserung war zu erkennen über die nächsten Monate. Nach Absetzen der Therapie trat zuerst keinerlei Veränderung auf. Erst nach einigen Wochen trat erste Symptomatik wieder auf, diese verstärkte sich dann innerhalb von Monaten, allerdings nicht zurück auf das Ausgangsniveau. Teilweise bleiben die Patienten über Jahre beschwerdefrei.

In der Folgezeit wurden in nicht öffentlichen Studien weitere Patienten mit RBD mit Melatonin behandelt. Die obigen Befunde konnten bestätigt werden. Zuletzt wurde eine kontrollierte klinische Studie durchgeführt. Hier zeigte sich Melatonin statistisch signifikant überlegen gegenüber Placebo. Darüber hinaus konnte der überdauernde Effekt der Melatonin-Therapie bestätigt werden. Es ist bekannt, dass bei Patienten mit Parkinsonscher Erkrankung die striatale dopaminerge Innervation reduziert ist. Diese Reduktion kann zumeist bereits vor Auftreten klinischer Symptomatik in der funktionellen Bildgebung mittels Dopamin-Transporter-Liganden (DaTSCAN) aufgezeigt werden. Die bestehende Literatur zeigt an, dass bei Patienten mit RBD ebenfalls eine reduzierte Dopamin-Transporter-Dichte im Striatum vorliegt. Dies wird als Beleg genommen, dass RBD ein früher Prädiktor von Alpha-Synukleinopathien ist.

In der Zwischenzeit wurde bei 12 Patienten mit RBD nach einem Jahr Melatoninbehanldung ein erneutes DaTSCAN durchgeführt. Bei der Hälfte der Patienten zeigte sich ein deutlicher Anstieg der Dopamintransporterdichte im Striatum. Dieser Befund gibt einen weiteren Hinweis auf die protektive Wirkung von Melatonin in der Entstehung von Synukleinopathien.

Die eigenen Befunde des Erfinders (s.o.) weisen nun darauf hin, dass Patienten mit klinischer RBD unter einer Melatonin-Behandlung keine verringerte Dopamin-Transporter-Dichte aufweisen. Der Befund lässt vermuten, dass Melatonin einen ursächlichen Einfluss auf RBD sowie Alpha-Synukleinopathien aufweist.

Zentral in der Behandlung mit Melatonin ist ein besonderes zeitliches Therapieschema.

Folgende Grundsätze sind aus unseren Beobachtungen und Untersuchungen abzuleiten:
- Exogenes Melatonin ist wirksam in der Behandlung der REM-Schlafverhaltensstörung;
- Exogenes Melatonin - eingesetzt bei Patienten mit RBD - reduziert die Konversionsrate zu Alpha-Synukleinopathien;
- Exogenes Melatonin verhindert die Entwicklung von Alpha-Synukleinopathien.

Melatonin muss gemäß vorliegender Erfindung nach folgendem Schema eingenommen werden:
1. Einnahmezeitpunkt ca. 30 Minuten vor der habituellen Zubettgehzeit;
2. Der Zubettgehzeitpunkt muss dem jeweiligen Chronotypus angepasst werden - beim Normotypus (Zubettgehzeitpunkt zwischen 22:00 und 24:00 Uhr) muss die Melatonin-Einnahme 30 Minuten vor dem Zubettgehzeitpunkt erfolgen. Beim Früh-bzw. Spättypen wird der Einnahmezeitpunkt angepasst;
3. Der Einnahmezeitpunkt von Melatonin muss eingehalten werden. Eine Verschiebung von 30 Minuten nach vorne oder hinten ist möglich. Eine Einnahme außerhalb dieses Zeitkorridors darf nicht erfolgen. In diesem Fall muss die Melatonin-Einnahme an diesem Abend unterbleiben.

Die Verabreichungsmenge ist unkritisch. Die Dosierung kann wahlfrei bestimmt werden. Im Rahmen der vorliegenden Erfindung hat sich herausgestellt, dass Melatonin in erster Linie als Zeitgeber wirkt. Es besteht die Vorstellung, dass die vorliegende Erfindung ein System schafft, mit dem "an ein Pendel einer Uhr angestoßen" wird. Daher ist für die vorliegende Erfindung auch der Einnahmezeitpunkt wichtig, wie er vorstehend definiert ist. Es besteht die Vorstellung, dass ein Abweichen von immer dem gleichen Zeitpunkt (innerhalb der oben angegebenen Toleranzen) dazu führt, dass "das Pendel der Uhr" stehen bleibt.

Nach der oben genannten kontrollierten Studie hat der Erfinder ca. 100 RBD-Patienten behandelt. Bei fast allen Patienten wurde die Symptomatik verbessert. Alle publizierenden Arbeitsgruppen der Welt verwenden in erster Linie Clonazepam, um diese Symptomatik zu verbessern.

Bei allen bisher berichteten, mit Clonazepam behandelten Patienten tritt die Symptomatik nach Absetzen wieder auf. Auch die Konversionsdaten von 80 - 100 Prozent aller RBD-Patienten basiert auf mit Clonazepam behandelten Patienten. Die Hälfte der vom Erfinder mit Melatonin behandelten Patienten setzt Melatonin nach 6 - 9 Monaten ab und lebt über Jahre ohne Symptome weiter.

Bestätigt wird der beschriebene Sachverhalt durch eine DaTSCAN-Untersuchung. Das ist eine hoch sensitive Untersuchungsmethode. In der Literatur berichtete Serienuntersuchungen bei Patienten wie bei Gesunden haben fast ausschließlich Abnahmen der Transporterdichte mit zunehmendem Alter gezeigt. Wichtig: sowohl inter- als auch intraindividuell. Auch nur sehr geringe Anstiege der Transporterdichte über die Zeit sind eine extreme Seltenheit.

Der erste Patient mit einem RBD - seit 2011 mit Melatonin behandelt -, den wir im DaTSCAN seriell untersucht haben (2011, 2013, 2015) zeigt einen kontinuierlichen, erheblichen Anstieg der Dopamintransporterdichte in allen untersuchten Regionen. Das heißt, einen zweimaligen Anstieg. Kein einziger in der Weltliteratur beschriebener Proband oder Patient hat einen auch nur einmaligen Anstieg dieser Größenordnung. Der DaTSCAN-Befund 2011 wird mit "sicheres Vorliegen einer Parkinson-Erkrankung" diagnostiziert, der Befund 2013 mit "Grenzbefund" und der Befund von 2015 mit "kein Anhalt für das Vorliegen einer Parkinson-Erkrankung".

## Patentansprüche

1. Exogenes Melatonin zur Verwendung bei einer Behandlung einer REM-Schlafverhaltensstörung eines Menschen mit einem individuellen Chronotypus, wobei ein Einnahmezeitpunkt einer Dosis des Melatonin einmal täglich bei einem Normotypus, dessen Zubettgehzeit zwischen 22:00 Uhr und 24:00 Uhr festgelegt ist, ca. 30 Minuten vor der habituellen Zubettgehzeit festgelegt wird,
**dadurch gekennzeichnet, dass** die habituelle Zubettgehzeit des Menschen an einem jeweils folgenden Tag in einem Zeitfenster von maximal 60 Minuten in Bezug zur habituellen Zubettgehzeit des Vortages festgelegt wird und ein Zeitkorridor für die Einnahme festgelegt wird, der eine Verschiebung des Einnahmezeitpunkts innerhalb des Zeitfensters ermöglicht, derart, dass der Zubettgehzeitpunkt an den individuellen Chronotypus des Menschen angepasst wird.

2. Exogenes Melatonin zur Verwendung bei einer Behandlung einer REM-Schlafverhaltensstörung eines Menschen nach Anspruch 1, **dadurch gekennzeichnet, dass** die Dosis Melatonin zur Behandlung der REM-Schlafverhaltensstörung auch die Entwicklung von Alpha-Synukleinopathien aus der neurodegenerativen -Erkrankungsgruppe verlangsamt.

3. Exogenes Melatonin zur Verwendung bei einer Behandlung einer REM-Schlafverhaltensstörung eines Menschen nach Anspruch 1 oder 2, **dadurch gekennzeichnet, dass** die Dosis Melatonin zur Behandlung der REM-Schlafverhaltensstörung auch die Entwicklung von Alpha-Synukleinopathien aus der Erkrankungsgruppe Lewy-Body-Demenz, multiple Systemmatropie und Parkinson verlangsamt.

4. Exogenes Melatonin zur Verwendung bei einer Behandlung einer REM-Schlafverhaltensstörung eines Menschen nach Anspruch 1 , **dadurch gekennzeichnet, dass** die Dosis Melatonin zur Behandlung der REM-Schlafverhaltensstörung die Entwicklung von mit Schlafstörungen verbundenen Alpha-Synukleinopathien aus der neurodegenerativen - Erkrankungsgruppe verhindert.

## Claims

1. Exogenous melatonin for use in a treatment of a REM sleep behaviour disorder in a human being with an individual chronotype, wherein a time for ingesting a dose of the melatonin once a day is set at approximately 30 minutes prior to the normal time for going to bed in the case of a normotype, whose time for going to bed is set at between 22:00 hours and 24:00 hours,
**characterized in that** the normal time for going to bed for the human beings on each subsequent day is set in a time window of a maximum of 60 minutes with respect to the normal time for going to bed of the previous day and a timeframe for the ingestion is set which enables the time for ingesting a dose to be shifted within the time window, such that the time for going to bed is adapted to the individual chronotype of the human being.

2. Exogenous melatonin for use in a treatment of a REM sleep behaviour disorder in a human being as claimed in claim 1, **characterized in that** the dose of melatonin for the treatment of the REM sleep behaviour disorder also delays the development of alpha-synucleinopathies from the neurodegenerative disorder group.

3. Exogenous melatonin for use in a treatment of a REM sleep behaviour disorder in a human being as claimed in claim 1 or claim 2, **characterized in that** the dose of melatonin for the treatment of the REM sleep behaviour disorder also delays the development of alpha-synucleinopathies from the dementia with Lewy bodies, multiple-system atrophy and Parkinson disease group.

4. Exogenous melatonin for use in a treatment of a REM sleep behaviour disorder in a human being as claimed in claim 1, **characterized in that** the dose of melatonin for the treatment of the REM sleep behaviour disorder inhibits the development of alpha-synucleinopathies linked to sleep disorders from the neurodegenerative disorder group.

## Revendications

1. Mélatonine exogène, destinée à être utilisée pour un traitement d'un trouble du comportement du sommeil MOR d'un individu d'un chronotype individuel, un moment de la prise d'une dose de la mélatonine étant fixé à une fois par jour pour un normotype, qui se couche entre 22 heures et 24 heures, environ 30 minutes avant l'heure habituelle à laquelle il se couche,
**caractérisée en ce que** l'heure habituelle du coucher de l'individu un jour respectivement suivant est fixée dans une plage horaire d'un maximum de 60 minutes en rapport à l'heure habituelle du coucher et un créneau temporel pour la prise est fixé, qui permet un décalage du moment de la prise dans la plage horaire, de telle sorte que le moment du coucher soit adapté au chronotype individuel de l'individu.

2. Mélatonine exogène destinée à être utilisée pour un traitement d'un trouble du comportement du sommeil MOR d'un individu selon la revendication 1, **caractérisée en ce que** la dose de mélatonine pour le traitement du trouble du comportement du sommeil MOR ralentit également le développement des alpha-synucléinopathies du groupe des maladies neurodégénératives.

3. Mélatonine exogène destinée à être utilisée pour un traitement d'un trouble du comportement du sommeil MOR d'un individu selon la revendication 1 ou 2, **caractérisée en ce que** la dose de mélatonine pour le traitement du trouble du comportement du sommeil MOR ralentit également le développement des alpha-synucléinopathies du groupe des maladies démence à corps de Lewy, atrophie multisystémique et Parkinson.

4. Mélatonine exogène destinée à être utilisée pour un traitement d'un trouble du comportement du sommeil MOR d'un individu selon la revendication 1 , **caractérisée en ce que** la dose de mélatonine pour le traitement du trouble du comportement du sommeil MOR empêche également le développement des alpha-synucléinopathies du groupe des maladies neurodégénératives liées aux troubles du sommeil.
